# FASCICULE DE BREVET EUROPEEN

(11) **EP 3 381 477 B1**
(45) Date de publication et mention de la délivrance du brevet: **18.12.2019**
(21) Numéro de dépôt: 18164423.8
(22) Date de dépôt: 27.03.2018
(51) Int. Cl.: A61L 2/26, B65D 81/00, A61J 1/00, B65B 55/00, A61B 50/00, A61M 5/00

(54) **CASSETTE DE STÉRILISATION POUR ARTICLES ASEPTISÉS, COMPORTANT UNE ENCEINTE PERFORÉE**
STERILISIERUNGSKASSETTE FÜR DESINFIZIERTE ARTIKEL, DIE EINEN PERFORIERTEN BEHÄLTER UMFASST
STERILISATION CASSETTE FOR ASEPTIC ITEMS, COMPRISING A PERFORATED ENCLOSURE

(30) Priorité: 27.03.2017 FR 1752528
(43) Date de publication de la demande: 03.10.2018
(73) Titulaire: Tekneuro, 64140 Billère (FR)
(72) Inventeur: CARRARA, Jean-Pierre, 69440 SAINT LAURENT D'AGNY (FR)
(74) Mandataire: Guérin, Jean-Philippe

(56) Documents cités:
- WO-A1-92/01514
- JP-A- H1 135 124
- US-A- 2 833 007

## Description

L'invention a trait au domaine de la production d'articles stériles pour, par exemple, l'industrie pharmaceutique ou médicale.

Dans ces industries, de nombreux articles sont aseptisés au cours de leur fabrication puis emballés dans un contenant qui maintient la stérilité jusqu'à l'utilisation. Il est ainsi garanti que les seringues ou autres instruments soient dépourvus de bactéries, virus, parasites, ou prions lors de leur utilisation.

Dans un tel environnement de production, les exigences en matière de sécurité du procédé sont des plus élevées. Toutes les opérations de la chaîne de production doivent garantir la stérilité effective des articles produits et le maintien de cette stérilité, en plus de garantir une fabrication conforme aux standards de qualité et sécurité de ces industries.

Dans ce contexte, les opérations de transfert d'un poste de production à un autre doivent être considérées avec autant d'attention que les opérations elles-mêmes réalisées sur les postes de travail. Par exemple, entre deux machines réalisant chacune une opération de la chaîne de production, il est nécessaire de transporter les articles en cours de réalisation. Que l'opération de transport se fasse manuellement ou de manière automatisée, elle doit garantir l'intégrité et la stérilité des articles produits.

Parmis les différentes opérations de la chaîne de production, l'opération de stérilisation des articles produits est critique à cet égard puisque le respect des plus hautes exigences de cette industrie doit être mis en œuvre aussi bien pour l'opération de stérilisation que pour l'acheminement en amont et en aval du poste de stérilisation. Or, les opérations de manutention entre les machines de différents postes de travail sont des opérations risquées pour l'intégrité des articles et de leur aseptisation.

On connait des solutions pour sécuriser la stérilisation d'articles aseptisés et leurs transferts. On emploie couramment des cages dans lesquelles on place par des portes les articles aseptisés à stériliser, les portes étant ensuite refermées et verrouillées. Ces cages sont ensuite mises directement dans des dispositifs de stérilisation tels que des autoclaves puis, en fin de stérilisation, ce sont les cages elles-mêmes qui sont déplacées jusqu'au poste de production suivant. Les portes des cages sont munies de barreaux et se referment sur les articles aseptisés pour les maintenir à l'intérieur et, lorsque les articles aseptisés doivent être déchargés des cages, les portes des cages sont déverrouillées puis ouvertes, manuellement ou automatiquement et le contenu de la cage est déchargé. Lorque l'ouverture est manuelle, l'opérateur doit manipuler un verrou et doit manuellement ouvrir la porte. Lorque l'ouverture est automatisée, la cage est positionnée face à un dispositif d'ouverture qui détecte la cage et commande un actionneur de déverrouillage de la porte puis commande l'ouverture de la porte. On connait les cassettes décrites dans les documents US 2,833,007 et WO 92/01514.

L'invention a pour but d'améliorer les dispositifs de stérilisation de l'art antérieur.

A cet effet, l'invention vise une cassette de stérilisation pour articles aseptisés, comportant une enceinte perforée, un support pour lesdits articles aseptisés, et un dispositif d'immobilisation desdits articles aseptisés à l'intérieur de l'enceinte perforée. Le support pour lesdits articles aseptisés comporte des glissières de suspension. Le dispositif d'immobilisation comporte une barrière mobile verticalement entre une position basse où elle obture les glissières de suspension et une position haute où elle libère les glissières de suspension, la barrière mobile étant associée à un doigt d'actionnement. La cassette de stérilisation comporte une rainure de déverrouillage sensiblement parallèle aux glissières de suspension, le doigt d'actionnement étant disposé dans la rainure de déverrouillage.

La cassette de stérilisation selon l'invention est apte à assurer un maintien adéquat des articles aseptisés durant la stérilisation et les phases de transfert, et garanti un déverrouillage simple et rapide de la barrière mobile, quand il est question de charger ou décharger les articles aseptisés. Le doigt d'actionnement étant disposé à l'intérieur de la rainure de déverrouillage, il est protégé de l'environnement extérieur et tout actionnement accidentel de la barrière mobile est empêché. Ceci permet l'emploi d'une barrière mobile simple pour laquelle la gravité est mise à profit pour le passage de la position haute à la position basse correspondant à la fermeture de la barrière mobile. Le passage de la position basse à la position haute, correspondant à l'ouverture de la barrière mobile, se fait en actionnant le doigt de commande, ce qui peut être fait facilement par un organe fixe s'insérant dans la rainure de déverrouillage. Un tel organe fixe peut être mis en œuvre lorsque la cassette est approchée d'un poste de chargement ou de déchargement, les glissières de suspension étant avancées contre un organe de chargement ou de déchargement et cette avancée peut donc être mise à profit pour l'ouverture de la barrière mobile par cet organe fixe grâce à la disposition de la rainure de déverrouillage qui est sensiblement parallèle aux glissières de suspension.

Une ouverture automatique de la barrière mobile est ainsi réalisée par des moyens mécaniques, plus fiables que les moyens employés habituellement pour l'automatisation, tels que cellules de détection de présence et actionneurs divers.

La simplicité du mécanisme d'ouverture et de fermeture de la barrière mobile permet de réduire le nombre de pièces mobiles et donc le coût des cassettes de stérilisation, tout en réduisant les frottements et augmentant la fiabilité.

La cassette de stérilisation peut comporter les caractéristiques additionnelles suivantes, seules ou en combinaison :
- la cassette de stérilisation comporte un deuxième doigt d'actionnement associé à la barrière mobile et disposé dans une deuxième rainure de déverrouillage, les deux rainures de déverrouillage étant disposées de part et d'autre de la barrière mobile ;
- l'enceinte perforée comporte un orifice d'accès pour le doigt d'actionnement, afin de permettre son montage et son démontage ;
- la cassette de stérilisation comporte un couvercle pour l'enceinte perforée, les glissières de suspension étant pratiquées dans ce couvercle, la barrière mobile pouvant être montée dans ce couvercle ;
- la barrière mobile est constituée d'un barreau de section rectangulaire, voire de section carrée ;
- la barrière mobile est montée glissante dans une empreinte constituée par des surfaces du couvercle.

Un autre objet de l'invention vise un kit de convoyage pour articles aseptisés comportant une cassette de stérilisation tel que décrite ci-dessus, une paire de dents présentant chacune une rampe, un guide d'assise, et des guides latéraux, le guide d'assise et les guides latéraux étant adaptés à guider la cassette de stérilisation de sorte que les dents pénètrent chacune dans une rainure de déverrouillage.

Le kit de convoyage peut comporter en outre un dispositif de chargement/déchargement, constitué de rampes longitudinales, sur lequel sont montées les dents. Le guide d'assise peut par ailleurs être incliné de sorte à former une pente en direction des dents, pour mettre à profit la gravité dans le déplacement des articles aseptisés entre la cassette de stérilisation et le dispositif de chargement/déchargement, dans un sens ou dans l'autre.

Un exemple préféré de réalisation de l'invention va maintenant être décrit en référence aux dessins annexés dans lesquels :
- la figure 1 est une vue en perspective d'une cassette de stérilisation selon l'invention ;
- la figure 2 représente la cassette de stérilisation de la figure 1, dépourvue de son couvercle ;
- la figure 3 est une vue de côté du couvercle de la cassette de stérilisation de la figure 1 ;
- la figure 4 est une vue de dessous, tronquée, du couvercle de la figure 3 ;
- la figure 5 est une vue de face du couvercle de la figure 3 ;
- la figure 6 représente la barrière mobile de la cassette de stérilisation de la figure 1 ;
- la figure 7 est une vue de détail de la figure 1 ;
- les figures 8 et 9 sont des vues de face de la cassette de stérilisation de la figure 1, la barrière mobile étant respectivement en position haute et en position basse ;
- la figure 10 représente en perspective un kit de convoyage pour articles aseptisés selon l'invention, mis en œuvre pour le déchargement d'une cassette de stérilisation ;
- les figures 11 à 15 montrent différentes étapes du déchargement de la cassette de stérilisation ;
- la figure 16 représente en perspective un kit de convoyage pour articles aseptisés selon l'invention, mis en œuvre pour le chargement d'une cassette de stérilisation ;
- les figures 17 et 18 montrent différentes étapes du chargement de la cassette de stérilisation.

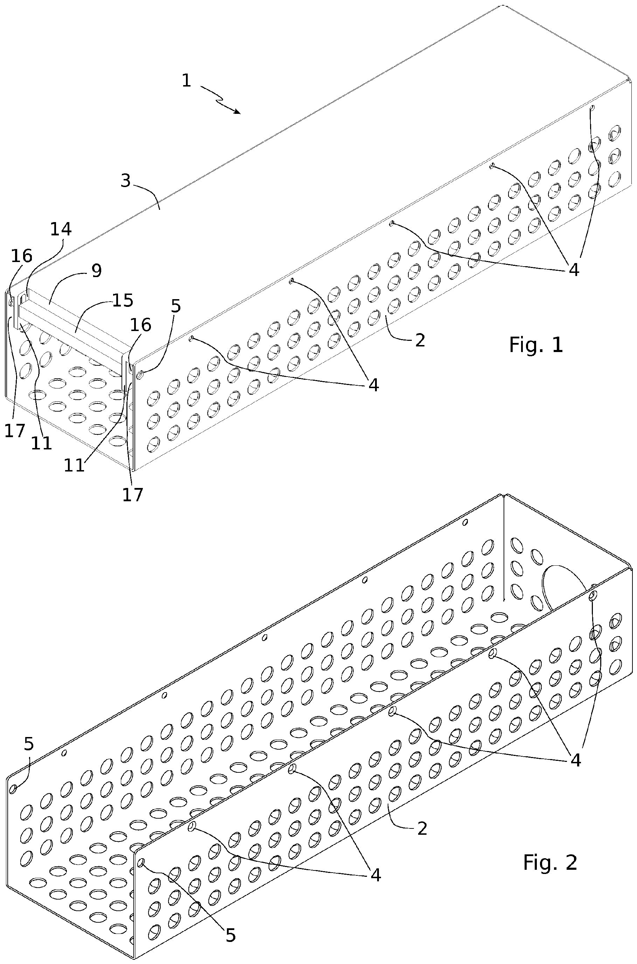

La figure 1 représente une cassette de stérilisation 1 adaptée à contenir des articles aseptisés durant leur procédé de fabrication qui inclut le passage par un poste de stérilisation et les manipulations avant et après ce poste.

Dans le présent exemple, la cassette de stérilisation 1 est prévue pour contenir des seringues en cours de fabrication. Ces cassettes 1 permettent de charger un lot de, par exemple, 6 lignes de 46 seringues, de les faire transiter entre les différents postes de travail de la ligne de production, ces postes de travail incluant un poste de stérilisation, voire même de les intégrer à la chaîne logistique en les faisant transiter d'un site de production à un autre ou entre sous-traitants si besoin.

La cassette de stérilisation comporte une enceinte perforée 2, un couvercle 3, et une barrière mobile 15.

La figure 2 montre l'enceinte perforée 2 seule. Celle-ci doit être réalisée dans un matériau qui est compatible avec l'industrie médicale et pharmaceutique, qui est résistant aux hautes températures mises en œuvre dans les procédés de stérilisation, et qui est apte à protéger les seringues qu'elle contient. Cette enceinte peut être réalisée, par exemple, à partir d'une tôle d'acier inoxydable de 1,5 mm d'épaisseur perforée puis pliée et soudée pour obtenir la pièce montrée à la figure 2. L'enceinte perforée comporte également des trous de montage 4 adaptés à recevoir les vis de montage du couvercle 3, ainsi que deux orifices d'accès 5 dont le rôle est expliqué plus loin.

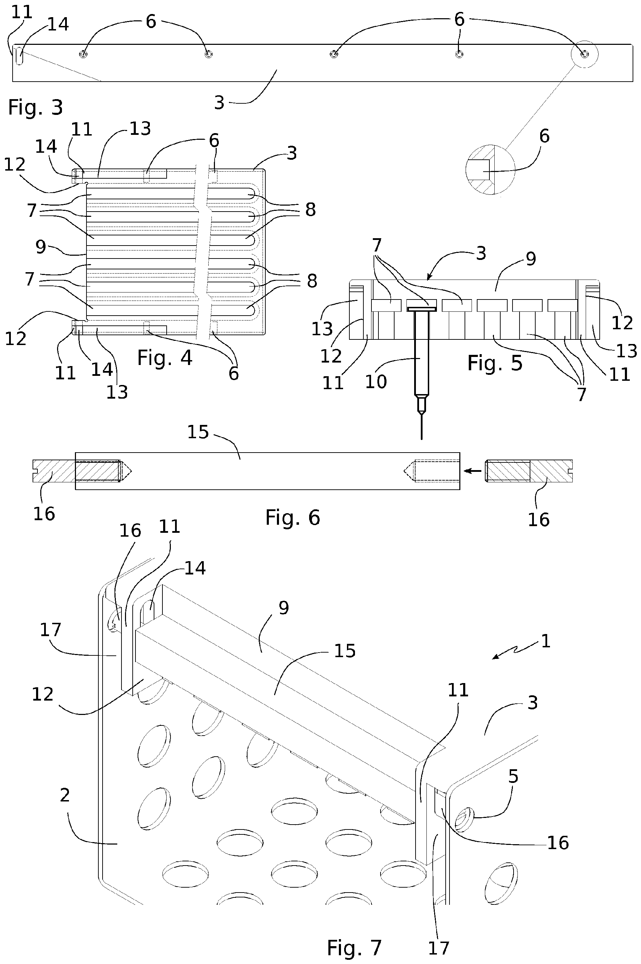

Les figures 3, 4, et 5 montrent le couvercle 3 vu, respectivement, de côté, de dessous, et de face. Le couvercle 3 est de préférence une pièce massive d'un matériau tel qu'un polymère compatible avec l'industrie médicale et pharmaceutique et ayant un faible coefficient de friction car les seringues seront amenées à glisser sur ce matériau. Il peut être réalisé par exemple en polyphénylsulfone par usinage, moulage ou un quelconque autre procédé.

Le couvercle 3 comporte des trous de fixation 6 taraudés et chanfreinés, pour son montage dans l'enceinte 2, et des glissières de suspension 7 s'étendant sur la longueur du couvercle 3. Les glissières de suspension 7 sont des rainures en forme de T qui s'achèvent par des demi cylindres 8 sur l'arrière du couvercle 3 et qui sont débouchants sur l'avant du couvercle 3, sur une face frontale 9 plane.

La figure 5 montre en exemple un cylindre de seringue 10 porté par une des glissières de suspension 7. Dans cet exemple, le cylindre de seringue 10 peut recevoir des accessoires tel qu'un portège aiguille. Il recevra également, plus loin dans le procédé de fabrication, un liquide à injecter et sera fermé par un piston de seringue. Le cylindre de seringue 10 est maintenu par sa garde dans la rainure en T, comme indiqué sur la figure 5, de sorte à être suspendu au couvercle 3 tout en pouvant coulisser le long de sa glissière 7.

La face frontale 9, sur laquelle débouchent les glissières de suspension 7, est encadrée par deux flasques 11 qui définissent deux surfaces latérales 12 planes et saillantes perpendiculairement à la face frontale 9, de part et d'autre de la sortie des glissières de suspension 7. Sur leur face opposée à la surface latérale, chaque flasque 11 comporte un évidemment 13 qui, dans le présent exemple, a une forme triangulaire. Chacun des flasques 11 comporte par ailleurs un trou oblong 14 qui définira la course de la barrière mobile 15.

La barrière mobile 15 est représentée vue de face à la figure 6. Il s'agit dans le présent exemple d'un barreau d'acier inoxydable de section carrée dont la longueur est égale à la distance entre les deux surfaces latérales 12 des flasques 11, au jeu de fonctionnement près. De même, le coté du carré définissant la section de la barrière mobile 15 est choisi conjointement à la position des trous oblongs de sorte que la barrière mobile vienne contre la face frontale 9, au jeu de fonctionnement près. La barrière mobile 15 est ainsi adaptée à être montée entre les flasques 11 de sorte qu'elle coulisse de haut en bas en glissant contre la face frontale 9 et les surfaces latérales 12.

Deux doigts d'actionnement 16 sont vissés sur les extrémités de la barrière mobile 15 et sont ici réalisés par des tétons filetés en acier inoxydable dont le diamètre correspond au diamètre des trous oblongs 14, au jeu de fonctionnement près. Les doigts d'actionnement 16 permettent ainsi de monter la barrière mobile de sorte qu'elle coulisse contre la face frontale 9 et les surfaces latérales 12, les doigts d'actionnement 16 coulissant eux dans les trous oblongs 14.

La vue de détail de la figure 7 montre la barrière mobile en place au sein du couvercle 3. Pour procéder à ce montage, le barreau constituant la barrière mobile 15 est inséré seul entre les surfaces latérales 12 de sorte à présenter les trous de fixation des doigts d'actionnement 16 en face des trous oblongs 14, puis à venir visser les doigts d'actionnement 16 dans la barrière mobile 15, à travers les orifices d'accès 5 de l'enceinte perforée 2.

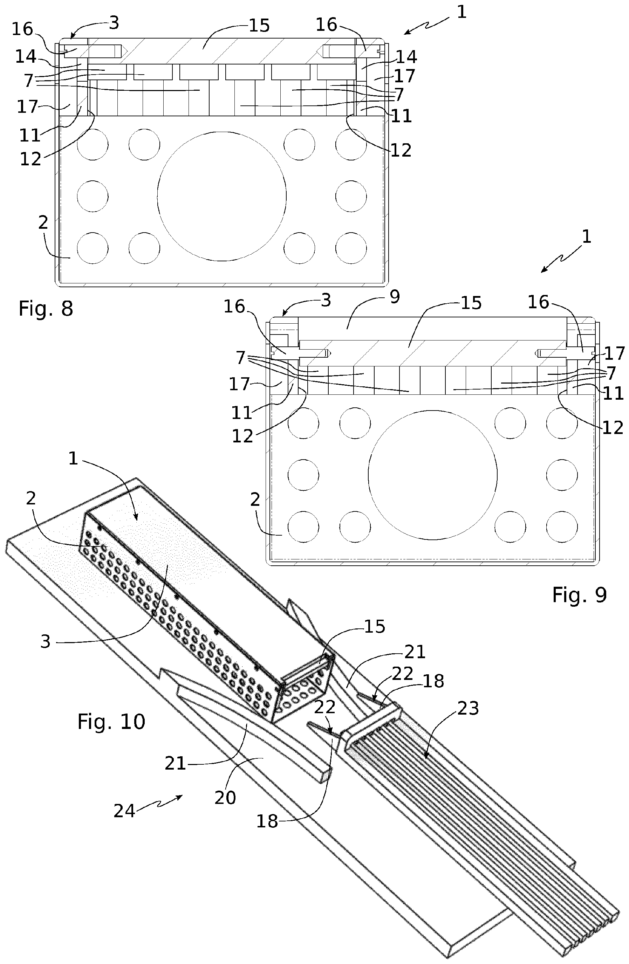

La longueur des doigts d'actionnement 16 est déterminée pour que chacun d'eux traverse le trou oblong 14 et dépasse le flasque 11 en venant se disposer dans l'évidement 13 correspondant, jusqu'à venir à proximité de l'enceinte perforée 2, sans la toucher. Les figures 8 et 9 représentent la cassette de stérilisation 1 vue de face, selon une coupe transversale passant par la barrière mobile 15 et montrent cette disposition des doigts d'actionnement 16.

Sur la figure 8, la barrière mobile 15 est en position haute et sur la figure 9, elle est en position basse.

Dans sa position haute (figure 8), la barrière mobile 15 libère les glissières de suspension 7, c'est à dire qu'elle n'entrave pas la sortie débouchante des glissières 7, permettant ainsi à des seringues qui seraient suspendues dans les glissières 7 de sortir, ou permettant au contraire à des seringues d'être chargées dans les glissières de suspension 7.

Dans sa position basse (figure 9), la barrière mobile 15 obture les glissières de suspension 7 c'est à dire que, dans le présent exemple, la barrière mobile 15 masque le haut des rainures en T qui forment les glissières 7 de sorte que les seringues suspendues par leur garde dans les glissières 7 viennent butter contre la barrière mobile 15 et sont ainsi verrouillées dans les glissières de suspension 7. De même, le chargement des glissières de suspension 7 en seringues depuis l'extérieur est empêché.

La manœuvre de la barrière mobile 15 entre ses positions haute et basse se fait par l'intermédiaire des doigts d'actionnement 16 qui sont disposés dans des rainures de déverrouillage 17 situées de part et d'autre de la barrière mobile 15. Chaque rainure de déverrouillage 17 est délimitée d'un côté par le flasque 11 et de l'autre côté par l'enceinte perforée 2. La forme interne de chaque rainure de déverrouillage 17 a par conséquent la forme de l'évidement 13 correspondant. Ces rainures de déverrouillage 17 sont parallèles à la direction dans laquelle s'étendent les glissières de suspension 7.

La cassette de stérilisation 1 qui vient d'être décrite est prévue pour s'intégrer dans une ligne de production qui comporte une étape de stérilisation. L'exemple décrit ci-après a pour but d'illustrer ce type de ligne de production pour exposer le rôle de la cassette de stérilisation. Selon cet exemple, il s'agit de produire des seringues pré-remplies selon le schéma suivant :
- production des seringues ;
- lavage des seringues ;
- stérilisation des seringues dans un autoclave ;
- remplissage des seringues avec le produit à injecter ;
- conditionnement des seringues finalisées en emballages stériles.

A partir de l'étape de stérilisation, toutes les étapes subséquentes doivent maintenir la stérilité des seringues jusqu'à l'emballage final.

Dans un tel exemple, la cassette de stérilisation 1 peut être employée après l'étape de lavage des seringues, à la sortie d'une machine ici dénommée « laveuse », jusqu'à l'étape de remplissage des seringues, à l'entrée d'une machine ici dénommée « remplisseuse ». Aussi bien la laveuse que la remplisseuse doivent maintenir les seringues par des moyens compatibles avec les glissières de suspension 7, c'est à dire que la laveuse doit disposer d'un dispositif de déchargement permettant de décharger les seringues dans les glissières de suspension 7, tandis que la remplisseuse doit disposer d'un dispositif de chargement permettant de charger les seringues à partir des glissières de suspension 7.

Une cassette de stérilisation 1 est donc présentée à la sortie de la laveuse jusqu'à ce que les glissières de suspension 7 viennent au contact du dispositif de déchargement de la laveuse, les seringues lavées sont ensuite transférées dans la cassette de stérilisation 1, puis la cassette est ensuite convoyée jusque dans l'autoclave qui procède à la stérilisation. En fin de stérilisation, la cassette est convoyée hors de l'autoclave pour être présentée à l'entrée de la remplisseuse jusqu'à ce que les glissières de suspension 7 viennent au contact du dispositif de chargement de la remplisseuse, et les seringues sont ensuite transférées dans la remplisseuse pour y être remplies.

La figure 10 représente un kit de convoyage 24 qui peut être avantageusement employé avec la cassette de stérilisation 1. Ce kit de convoyage 24 comporte, en plus de la cassette de stérilisation 1, deux dents 18 qui sont montées sur un dispositif de chargement/déchargement 23 coopérant avec la remplisseuse suivant l'exemple ici exposé. Ce dispositif de chargement/déchargement 23 est constitué de rampes longitudinales adaptées à prendre en charge les seringues pour le compte de la remplisseuse. Pour des raisons de clarté, le dispositif de chargement/déchargement 23 a été représenté seul, sans ses fixations à la remplisseuse.

La cassette de stérilisation 1, au sortir de l'autoclave, va donc être mise dans la configuration de la figure 10 en vue d'alimenter la remplisseuse. Le dispositif de chargement/déchargement 23 est donc ici utilisé en chargement.

Les dents 18 sont de forme triangulaire et définissent une rampe 22. L'écartement entre les deux dents 18 correspond à la distance entre les deux rainures de déverrouillage 17. Le kit de convoyage 24 comporte également un guide d'assise 20 ainsi que deux guides latéraux 21 qui sont dimensionnés et fixés par rapport au dispositif de chargement/déchargement 23 de sorte que, lorsqu'une cassette de stérilisation 1 est insérée entre les guides latéraux 21 en direction des dents 18, le positionnement en hauteur assuré par le guide d'assise 20 et le positionnement latéral assuré par les guides latéraux 21 garantit que les dents 18 s'insèrent dans les deux rainures de déverrouillage 17.

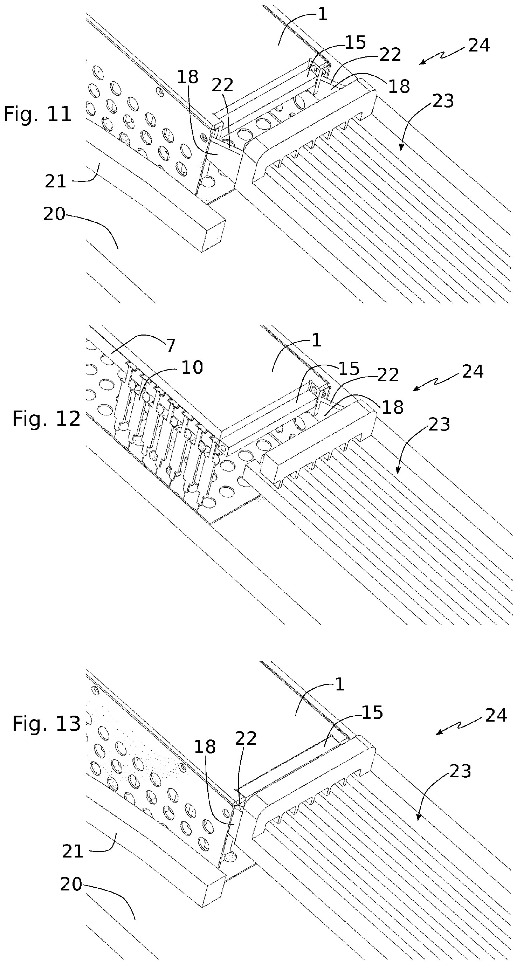

Le mouvement d'approche de la cassette de stérilisation 1 en direction du dispositif de chargement/déchargement 23 peut ainsi être avantageusement mis à profit pour manœuvrer les doigts d'actionnement 16 grâce aux rampes 22 des dents 18. Cette manœuvre est illustrée sur les figures 11 à 15.

La figure 11 illustre un moment où, la cassette de stérilisation 1 étant glissée le long du guide d'assise 20, elle arrive à proximité des dents 18 jusqu'à l'engagement de ces dernières dans les rainures de déverrouillage 17, les rampes 22 des dents 18 venant au contact des doigts d'actionnement 16.

La vue en coupe longitudinale de la figure 12 montre la position des seringues dans la configuration de la figure 11. La barrière mobile 15 est encore en position basse et les seringues sont verrouillées dans la cassette de stérilisation 1.

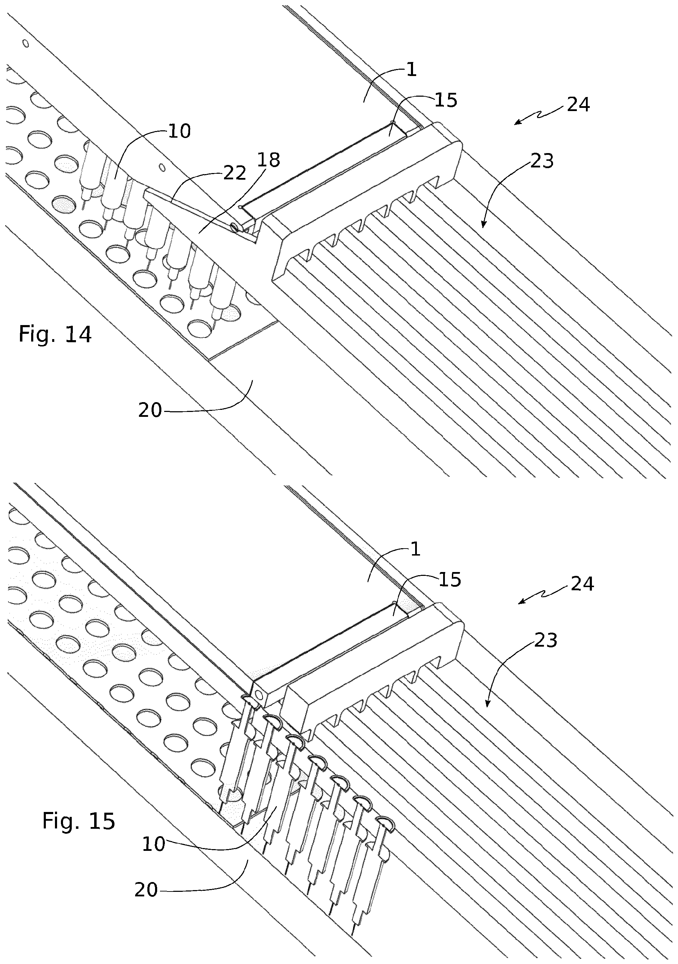

La figure 13 illustre le moment où la cassette de stérilisation 1 est avancée au maximum contre le dispositif de chargement/déchargement 23, la barrière mobile 15 ayant été soulevée par l'action des doigts d'actionnement 16 glissant sur les rampes 22 des dents 18 au fur et à mesure de l'avancée de la cassette de stérilisation 1. La vue en coupe longitudinale de la figure 14 montre les positions relatives du couvercle 3 et des dents 18 dans la configuration de la figure 13.

Dans cette configuration, la cassette de stérilisation 1 est en buttée contre le dispositif de chargement/déchargement 23 de sorte que les glissières de suspension 7 viennent contre le dispositif de chargement/déchargement 23. Grâce à l'ouverture de la barrière mobile 15, le passage des seringues 10 est alors possible entre les glissières de suspension 7 et le dispositif de chargement/déchargement 23.

Dans cet exemple de déchargement de la cassette de stérilisation 1, illustré aux figures 11 à 15, la gravité est mise à profit pour entraîner le glissement des seringues 10 de la cassette 1 au dispositif de chargement/déchargement 23, et ce en inclinant le guide d'assise 20 comme montré dans ces figures.

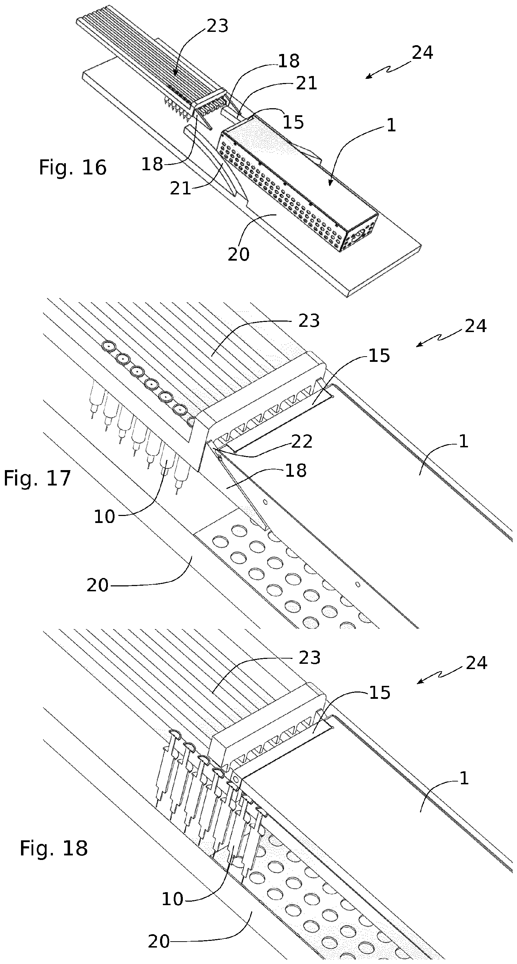

De manière complémentaire, la cassette de stérilisation 1 peut être chargée de seringues suivant un exemple relatif aux figures 16 à 18. Le chargement de la cassette de stérilisation 1 peut être réalisé avec un guide d'assise 20 incliné selon une pente ascendante en direction d'un dispositif de chargement/déchargement 23 coopérant avec la laveuse, que l'on souhaite décharger, comme illustré à la figure 16. De même que sur les figures précédentes, seul le dispositif de chargement/déchargement 23 a été représenté, sans ses fixations à la laveuse.

La cassette de stérilisation 1, avant de partir pour l'autoclave, va donc être mise dans la configuration de la figure 16 en vue d'être chargée par la laveuse. Le dispositif de chargement/déchargement 23 est donc ici utilisé en déchargement. Dans cette configuration, la laveuse dispose avantageusement de moyens de blocage des seringues 10, tels que des doigts de blocage au niveau de chaque ligne de seringue 10 que la laveuse peut piloter.

De la même manière que décrit précédemment pour le déchargement de la cassette de stérilisation 1, cette dernière est poussée contre le dispositif de chargement/déchargement 23 jusqu'à ce que les dents 18 soient engagées dans les rainures de déverrouillage 17 et que les doigts d'actionnement 16 soient soulevés par les rampes 22 des dents 18, jusqu'à la position de la figure 17. La vue en coupe longitudinale de la figure 17 montre la position maximale de la cassette de stérilisation 1 contre le dispositif de chargement/déchargement 23.

Les seringues 10 sont alors libres de glisser depuis le dispositif de chargement/déchargement 23 vers les glissières de suspension 7, chargeant ainsi la cassette de stérilisation 1.

Par ailleurs, en ce qui concerne les déplacements de la cassette de stérilisation 1 entre son chargement, les opérations de stérilisation, et son déchargement, elle peut être convoyée et approchée du dispositif de chargement/déchargement 23 de manière automatique (par exemple sur un tapis roulant ou par des actionneurs) ou manuelle. Les avantages liés au positionnement et à l'ouverture aisée et sûre sont les mêmes quel que soit le mode de convoyage de sorte que cette solution flexible peut être employée même dans une ligne de production mixte automatique/manuelle.

De même, la flexibilité est également de mise pour ce qui est du maintien de la stérilité tout au long de la chaîne de production dans la mesure où la cassette de stérilisation peut bien entendu évoluer en milieu stérile entre la sortie de l'autoclave et la remplisseuse, si la ligne de production est ainsi équipée, mais la cassette de stérilisation est également adaptée à être ensachée en emballage stérile pour une manipulation hors zone stérile de la ligne de production.

D'autres variantes de réalisation de la cassette stérile 1 et du kit de convoyage 24 peuvent être mises en œuvre sans sortir du cadre de l'invention. Par exemple, la description se réfère à des seringues ou cylindres de seringue mais l'invention est applicable à n'importe quel article aseptisé qui peut être suspendu. Les seringues 10 des figures 11 à 18 sont des seringues complètes (cylindre et piston) tandis que sur la figure 5, il s'agit simplement d'un cylindre de seringue, bien qu'ils portent tous la référence 10 par souci de simplification.

De même, l'évidement 13 peut être d'une quelconque forme autre que triangulaire, ainsi que la barrière mobile qui peut avoir une forme autre que celle décrite en exemple. Cette dernière peut par ailleurs être constituée d'une seule pièce avec les doigts d'actionnement. Le guide d'assise 20 et les guides latéraux 21, quant à eux, peuvent comporter des formes très diverses, autres que celles décrites, à partir du moment où ils remplissent leurs fonctions de guidage et de positionnement.

## Revendications

1. Cassette de stérilisation (1) pour articles aseptisés (10), comportant une enceinte perforée, un support pour lesdits articles aseptisés, et un dispositif d'immobilisation desdits articles aseptisés à l'intérieur de l'enceinte perforée, **caractérisée en ce que** : le support pour lesdits articles aseptisés comporte des glissières de suspension (7) ; **en ce que** le dispositif d'immobilisation comporte une barrière mobile (15) verticalement entre une position basse où elle obture les glissières de suspension (7) et une position haute où elle libère les glissières de suspension (7), la barrière mobile (15) étant associée à un doigt d'actionnement (16) ; et **en ce que** la cassette de stérilisation (1) comporte une rainure de déverrouillage (17) sensiblement parallèle aux glissières de suspension (7), le doigt d'actionnement (16) étant disposé dans la rainure de déverrouillage (17).

2. Cassette de stérilisation selon la revendication 1, **caractérisée en ce qu'**elle comporte un deuxième doigt d'actionnement (16) associé à la barrière mobile (15) et disposé dans une deuxième rainure de déverrouillage (17), les deux rainures de déverrouillage (17) étant disposées de part et d'autre de la barrière mobile (15).

3. Cassette de stérilisation selon l'une des revendications 1 ou 2, **caractérisée en ce que** l'enceinte perforée (2) comporte un orifice d'accès (5) pour le doigt d'actionnement (16).

4. Cassette de stérilisation selon l'une des revendications 1 à 3, **caractérisée en ce qu'**elle comporte un couvercle (3) pour l'enceinte perforée (2), les glissières de suspension (7) étant pratiquées dans ce couvercle (3).

5. Cassette de stérilisation selon la revendication 4, **caractérisée en ce que** la barrière mobile (15) est montée dans ledit couvercle (3).

6. Cassette de stérilisation selon la revendication 5, **caractérisée en ce que** la barrière mobile (15) est constituée d'un barreau de section rectangulaire.

7. Cassette de stérilisation selon la revendication 6, **caractérisée en ce que** la barrière mobile (15) est montée glissante dans une empreinte constituée par des surfaces (9,12) du couvercle (3).

8. Kit de convoyage (24) pour articles aseptisés (10) comportant une cassette de stérilisation (1) selon l'une des revendications 2 à 7, une paire de dents (18) présentant chacune une rampe (22), un guide d'assise (20), et des guides latéraux (21), le guide d'assise (20) et les guides latéraux (21) étant adaptés à guider la cassette de stérilisation (1) de sorte que les dents (18) pénètrent chacune dans une rainure de déverrouillage (17).

9. Kit de convoyage selon la revendication 8, **caractérisé en ce qu'**il comporte en outre un dispositif de chargement/déchargement (23), constitué de rampes longitudinales, sur lequel sont montées les dents (18).

10. Kit de convoyage selon l'une des revendications 8 ou 9, **caractérisé en ce que** le guide d'assise (20) est incliné de sorte à former une pente en direction des dents (18).

## Patentansprüche

1. Sterilisationskassette (1) für desinfizierte Artikel (10), umfassend ein perforiertes Gehäuse, eine Halterung für die desinfizierten Artikel und eine Vorrichtung zum Immobilisieren der desinfizierten Artikel innerhalb des perforierten Gehäuses, **dadurch gekennzeichnet, dass**: die Halterung für die desinfizierten Artikel Aufhängeschlitten (7) umfasst; dass die Immobilisationsvorrichtung eine bewegliche Barriere (15) vertikal zwischen einer niedrigen Position, in der sie die Aufhängeschlitten (7) verschließt, und einer hohen Position, in der sie die Aufhängeschlitten (7) freigibt, umfasst, wobei die bewegliche Barriere (15) mit einem Betätigungsfinger (16) assoziiert ist; und dass die Sterilisationskassette (1) eine Entriegelungsnut (17) im Wesentlichen parallel zu den Aufhängeschlitten (7) aufweist, wobei der Betätigungsfinger (16) in der Entriegelungsnut (17) angeordnet ist.

2. Sterilisationskassette nach Anspruch 1, **dadurch gekennzeichnet, dass** sie einen zweiten Betätigungsfinger (16) umfasst, der mit der beweglichen Barriere (15) assoziiert und in einer zweiten Entriegelungsnut (17) angeordnet ist, wobei die zwei Entriegelungsnuten (17) auf beiden Seiten der beweglichen Barriere (15) angeordnet sind.

3. Sterilisationskassette nach einem der Ansprüche 1 oder 2, **dadurch gekennzeichnet, dass** das perforierte Gehäuse (2) eine Zugangsöffnung (5) für den Betätigungsfinger (16) aufweist.

4. Sterilisationskassette nach einem der Ansprüche 1 bis 3, **dadurch gekennzeichnet, dass** sie eine Abdeckung (3) für das perforierte Gehäuse (2) umfasst, wobei die Aufhängeschlitten (7) in dieser Abdeckung (3) angebracht sind.

5. Sterilisationskassette nach Anspruch 4, **dadurch gekennzeichnet, dass** die bewegliche Barriere (15) in der Abdeckung (3) montiert ist.

6. Sterilisationskassette nach Anspruch 5, **dadurch gekennzeichnet, dass** die bewegliche Barriere (15) aus einem Stab mit rechteckigem Querschnitt besteht.

7. Sterilisationskassette nach Anspruch 6, **dadurch gekennzeichnet, dass** die bewegliche Barriere (15) verschiebbar in einer Vertiefung montiert ist, die durch Oberflächen (9, 12) der Abdeckung (3) gebildet ist.

8. Beförderungs-Kit (24) für desinfizierte Artikel (10), umfassend eine Sterilisationskassette (1) nach einem der Ansprüche 2 bis 7, ein Paar Zähne (18), die jeweils eine Rampe (22), eine Sitzführung (20) und seitliche Führungen (21) aufweisen, wobei die Sitzführung (20) und die seitlichen Führungen (21) angepasst sind, um die Sterilisationskassette (1) auf eine Weise zu führen, dass die Zähne (18) jeweils eine Entriegelungsnut (17) eindringen.

9. Beförderungs-Kit nach Anspruch 8, **dadurch gekennzeichnet, dass** es ferner eine Be- und Entladevorrichtung (23) umfasst, die aus Längsrampen besteht, auf denen die Zähne (18) montiert sind.

10. Beförderungs-Kit nach einem der Ansprüche 8 oder 9, **dadurch gekennzeichnet, dass** die Sitzführung (20) geneigt ist, sodass sie eine Neigung in Richtung der Zähne (18) bildet.

## Claims

1. Sterilisation cassette (1) for sanitised articles (10), comprising a perforated enclosure, a support for said sanitised articles and a device for immobilising said sanitised articles inside the perforated enclosure, **characterised in that**: the support for said sanitised articles comprises suspension slides (7), **in that** the immobilisation device comprises a barrier (15) that is vertically movable between a low position, in which it blocks the suspension slides (7), and a high position, in which it releases the suspension slides (7), the movable barrier (15) being connected to an activation finger (16), and **in that** the sterilisation cassette (1) comprises an unlocking groove (17) substantially parallel to the suspension slides (7), the activation finger (16) being disposed in the unlocking groove (17).

2. Sterilisation cassette according to claim 1, **characterised in that** it comprises a second activation finger (16) connected to the movable barrier (15) and disposed in a second unlocking groove (17), the two unlocking grooves (17) being disposed on either side of the movable barrier (15).

3. Sterilisation cassette according to any of claims 1 or 2, **characterised in that** the perforated enclosure (2) comprises an access orifice (5) for the activation finger (16).

4. Sterilisation cassette according to any of claims 1 to 3, **characterised in that** it comprises a cover (3) for the perforated enclosure (2), the suspension slides (7) being made in this cover (3).

5. Sterilisation cassette according to claim 4, **characterised in that** the movable barrier (15) is mounted in said cover (3).

6. Sterilisation cassette according to claim 5, **characterised in that** the movable barrier (15) is formed by a rectangular section bar.

7. Sterilisation cassette according to claim 6, **characterised in that** the movable barrier (15) is slidably mounted in a recess formed by surfaces (9, 12) of the cover (3).

8. Conveying kit (24) for sanitised articles (10) comprising a sterilisation cassette (1) according to any of claims 2 to 7, a pair of teeth (18) each having a ramp (22), a support guide (20) and lateral guides (21), the support guide (20) and the lateral guides (21) being adapted to guide the sterilisation cassette (1) so that the teeth (18) each penetrate into an unlocking groove (17).

9. Conveying kit according to claim 8, **characterised in that** it further comprises a loading/unloading device (23), formed by longitudinal ramps, on which device the teeth (18) are mounted.

10. Conveying kit according to any of claims 8 or 9, **characterised in that** the support guide (20) is inclined so as to form a slope towards the teeth (18).
